# EUROPÄISCHE PATENTSCHRIFT

(11) **EP 1 529 768 B1**
(45) Veröffentlichungstag und Bekanntmachung des Hinweises auf die Patenterteilung: **03.10.2007**
(21) Anmeldenummer: 04025481.5
(22) Anmeldetag: 27.10.2004
(51) Int. Cl.: C07C 29/16, C07C 31/27, C07C 45/50, C07C 47/347, C07C 47/445

(54) **Verfahren zur Herstellung von TCD-Alkohol DM**
Process for the preparation of TCD-alcohol DM
Procédé de préparation de TCD-alcool DM

(30) Priorität: 08.11.2003 DE 10352260
(43) Veröffentlichungstag der Anmeldung: 11.05.2005
(73) Patentinhaber: OXEA Deutschland GmbH, 46147 Oberhausen (DE)
(72) Erfinder: Lappe, Peter, Dr., 46539 Dinslaken (DE); Springer, Helmut, 46539 Dinslaken (DE); Lukas, Rainer, Dr., 45133 Essen (DE)
(74) Vertreter: Szameitat, Jürgen

(56) Entgegenhaltungen:
- EP-A- 0 186 075
- EP-A- 0 811 424
- EP-A- 1 065 194
- DE-A- 2 928 313
- DATABASE CAPLUS CHEMICAL ABSTRACTS SERVICE, COLUMBUS, OHIO, US; XP002306531 gefunden im STN Database accession no. 1999:236498 & JP 11 100339 A (KURARAY CO LTD) 13. April 1999 (1999-04-13)
- DATABASE CAPLUS CHEMICAL ABSTRACTS SERVICE, COLUMBUS, OHIO, US; XP002306410 gefunden im STN Database accession no. 1981:514918 & JP 56 030938 A (MITSUBISHI PETROCHEMICAL CO LTD) 28. März 1981 (1981-03-28)

## Beschreibung

Die vorliegende Erfindung betrifft ein Verfahren zur Herstellung von TCD-Alkohol DM {3(4), 8(9)-Dihydroxymethyl-tricyclo[5.2.1.0^{2.6}]decan} aus Dicyclopentadien (DCP).

Das durch Dimerisierung von Cyclopentadien leicht zugängliche und auch großtechnisch hergestellte Dicyclopentadien (DCP) lässt sich zu anwendungstechnisch wichtigen Verbindungen umsetzen, denen das Tricyclodecan-Gerüst besondere Eigenschaften verleiht. Die vom DCP-abgeleiteten Verbindungen mit Tricyclodecan-Struktur werden in der Literatur häufig unterschiedlich bezeichnet. In Anlehnung an die aus Chemiker-Zeitung, 98, 1974, Seiten 70 bis 76 bekannte Nomenklatur für DCP-Derivate, wird auch im folgenden die auf dem Tricyclodecan-Gerüst, auch TCD-Gerüst genannt, aufbauende Nomenklatur verwendet.

TCD-Alkohol DM {3(4), 8(9)-Dihydroxymethyl-tricycIo[5.2.1.0^{2.6}]decan} besitzt als wichtiges Zwischenprodukt für die chemische Industrie eine große wirtschaftliche Bedeutung. Der zweiwertige Alkohol ist in vielfältiger Weise für unterschiedliche Anwendungen von hohem technischen Interesse: Acrylsäureester bzw. Methacrylsäureester von OH-gruppenhaltigen tricyclischen Decanolen (DE 22 00 021), als Bestandteil von bei Sauerstoffausschluss erhärtenden Acrylsäureesterklebstofien, (Meth)acrylsäureester von Ethergruppen enthaltenden tricyclischen Decanolen (EP 23 686), zur Herstellung von Kleb- und Dichtungsstoffen, Ester und Polyester der Tricyclodecanreihe (DE 934 889), die als Weichmacher und hochwertige Esterschmieröle geeignet sind, Riechstoffkompositionen (DE-OS 2 307 627) und säuresterilisationsfeste Polyesterlacke (DE 31 34 640) im Metall-Lackierungsbereich. TCD-Alkohol DM erhält man durch Hydrierung der Hydroformylierungsprodukte des Dicyclopentadiens, der sogenannten TCD-Aldehyde.

Die Herstellung von Aldehyden durch katalytische Anlagerung von Kohlenmonoxid und Wasserstoff an olefinische Doppelbindungen ist bekannt. Während diese Umsetzung früher nahezu ausschließlich mit Co als Katalysator durchgeführt wurde, arbeiten moderne Verfahren mit metallischem Rhodium oder mit Rhodiumverbindungen als Katalysatoren, die allein oder mit komplexbildenden Liganden, z.B. organischen Phosphinen oder Estern der phosphorigen Säure, eingesetzt werden. Unter den Reaktionsbedingungen als Katalysator wirksam sind nach übereinstimmender Auffassung der Fachwelt Hydridocarbonylverbindungen des Rhodiums, die sich durch die allgemeine Formel H[Rh(CO)₄₋ₓLₓ] wiedergeben lassen, wobei L einen Liganden bezeichnet und x gleich 0 oder eine ganze Zahl von 1 bis 3 ist.

Einen Sonderfall stellt die Hydroformylierung von Dienen dar. Während bei der Hydroformylierung konjugierter Diene unter den üblichen Bedingungen der Oxo-Synthese fast ausschließlich Monoaldehyde erhalten werden, lassen sich aus Dicyclopentadien (DCP) mit seinen isolierten Doppelbindungen neben den Mono- auch die Disubstitutionsprodukte gewinnen. Wegen der Gefahr der Retro-Diels-Alder-Reaktion bei den Temperaturen der Oxo-Synthese und der damit verbundenen Freisetzung von Cyclopentadien, das zur Komplexbildung gegenüber Übergangsmetallen befähigt ist und das die Aktivität der eingesetzten Katalysatoren mindern kann, muss die Hydroformylierung unter besonderen Bedingungen ablaufen. Es erwies sich als vorteilhaft, den früher üblichen Co-Katalysator durch Rhodium zu ersetzen, das eine hohe Selektivität der Umsetzung zu Aldehyden erreichen lässt und die Hydroformylierung bei Bedingungen erlaubt, bei denen das Ausmaß der Retro-Diels-Alder-Spaltung geringer ist. Eine zusammenfassende Darstellung über die Hydroformylierung von Dicyclopentadien und über die Weiterverarbeitung der TCD-Aldehyde findet sich in Chemiker-Zeitung 98, 1974, 70-76. Besondere Bedeutung besitzt dabei 8(9)-Formyl-tricyclo[5.2.1.0^{2.6}]dec-3-en, auch als TCD-Monenal bezeichnet und 3(4),8(9)-Bisformyl-tricyclo[5.2.1.0^{2.6}]decan, auch als TCD-Dialdehyd bezeichnet Wegen ihrer thermischen Labilität, die bei der Destillation zu Verlusten führt, werden die TCD-Aldehyde zumeist nicht rein isoliert, sondern als Rohprodukte der Hydroformylierungsreaktion weiterverarbeitet.

Aufgrund der vielseitigen Anwendungsmöglichkeiten besitzt TCD-Alkohol-DM ein hohes wirtschaftliches Interesse und in der Patentliteratur finden sich auch zahlreiche Hinweise auf Verfahren zu seiner Herstellung.

Das US-Patent US 4 647 708 beschreibt die Hydroformylierung von Dicyclopentadien mit Rh als Katalysator in Gegenwart von Ionenaustauschern (Dowex® MWA-1) in Toluol / THF als Lösungsmitteln. Die Umsetzung erfolgt bei 120°C und 27,5 MPa CO/H₂ (im Verhältnis 1 : 2) in zwei getrennten kontinuierlich betriebenen Autoklaven. Anhand der offenbarten Versuchsergebnisse kann man ersehen, dass die Ausbeute an TCD-Alkohol DM in dem 30 tägigen Versuchslauf von 85 % auf 65 % abfällt. Das Reaktionssystem ist somit für eine technische Anwendung nicht geeignet.

Im US-Patent US 4 262 147 wird der Einsatz von bimetallischen Rh/Co-Clustern auf Harzen wie Amberlite® IRA-68 beschrieben. Unter den angewandten Bedingungen (110°C, 11 MPa, 8 Stunden) wird eine Selektivität von 68 % an TCD-Alkohol DM in dieser einstufigen Synthese erhalten.

Ein modifiziertes Co-Verfahren wird in der DE-PS 3 008 497 beschrieben, wobei Dicyclopentadien unter Katalyse von Co/Tri-n-octylphosphin bei 200°C und einem Synthesegasdruck von 15 MPa umgesetzt wird. Nach einer Reaktionszeit von 5 Stunden wird der TCD-Alkohol DM in einer Ausbeute von 69 % erhalten. Als Nebenprodukte entstehen 11,7 % des TCD-Monoalkohols und 14,6 % Hydroxymethylcyclopentan. Aufgrund der notwendigerweise angewandten hohen Temperaturen kommt es zur Retro-Diels-Alder-Reaktion von Dicyclopentadien in Cyclopentadien und somit zu der Bildung signifikanter Mengen an Hydroxymethylcyclopentan. Daher ist diese Verfahrensvariante für eine technische Anwendung nicht geeignet.

Aus JP 111 00 339 ist bekannt, die Hydroformylierung von DCP in Isopropanol/Toluol mit Rhodiumdicarbonylacetylacetonat, Tris(2,4-di-tert.-butylphenyl)-phosphit und Triethylamin bei 120°C unter 8,8 MPa Synthesegas über 8 Stunden durchzuführen. Es werden 93 % TCD-Dialdehyd erhalten, der in Isopropanol bei 110°C und 0,78 MPa Wasserstoff für 6 Stunden an Raney-Nickel zu 91 % TCD-Alkohol DM hydriert wird. Der Einsatz der komplexen und aufwendig herstellbaren Phosphit-Liganden ist für eine technische Anwendung und auch aus wirtschaftlichen Gründen von Nachteil. Zudem wird die breite Verwendung von Phosphit-Liganden durch ihre im Vergleich zu konventionellen Phosphin-Liganden geringere Stabilität und höhere Hydrolyseempfindlichkeit gegenüber Wasser und -Säurespuren eingeschränkt und die während des kontinuierlich betriebenen Hydroformylierungsprozesses gebildeten Phosphonigsäuren beeinträchtigen die Katalysatorlebensdauer und müssen aufwendig aus dem Prozess entfernt werden. Außerdem ist bei der Verwendung von Aminen stets mit einer Verunreinigung des TCD-Alkohol DM mit stickstoffhaltigen Komponenten zu rechnen.

JP 560 30 938 beschreibt ein einstufiges Hydroformylierungsverfahren von Dicyclopentadien zum TCD-Dialdehyd in Gegenwart von Rhoidum-Phosphin-Katalysatoren. Es wird von einem vollständigen Umsatz an Dicylopendien bei einer Selektivität von 92,2% an TCD-Dialdehyd berichtet.

In der EP 1 065 194 wird ein Niederdruckverfahren zur Hydroformylierung von Dicyclopentadien beschrieben, in dem als Katalysatorsystem ebenfalls Rh/Tris-(2,4-di-tert.-butylphenyl)-phosphit eingesetzt wird. Die Hydroformylierung wird bei Drücken von 1 - 15 MPa und Temperaturen von 80 - 140°C durchgeführt. Als Lösungsmittel werden inerte Kohlwasserstoffe wie Toluol, Diisopropylbenzol oder Methylcyclohexan verwendet. Die Aufarbeitung des Hydroformylierungsprodukts erfolgt durch eine mehrstufige Extraktion unter Verwendung von mehrwertigen Alkoholen wie z.B. Ethylenglykol, wobei der Zusatz von tertiären Aminen empfohlen wird. Nach der Extraktion liegt das Oxorohprodukt vorwiegend in der Alkoholphase vor, während sich geringe Anteile an Mono- und Dialdehyd sowie die Hauptmenge an Rhodium und Phosphin-Liganden in der Kohlenwasserstoffphase befinden. Es wird darauf hingewiesen, dass die Extraktion in absoluter Abwesenheit von Sauerstoff erfolgen muss. Der Einsatz von Extraktionsmitteln unter Zusatz von tertiären Aminen sowie die absolute Notwendigkeit der Abwesenheit von Sauerstoff erschweren die technische Durchführung dieses Verfahrens und beinhalten das Risiko der Verunreinigung von TCD-Alkohol DM mit Spuren von Aminen.

Die bekannten Verfahren zur Herstellung von TCD-Alkohol DM durch Hydroformylierung von Dicyclopentadien mit nachfolgender Hydrierung erfordern entweder die Anwesenheit von speziellen, in der Technik nicht verfügbaren oder umweltunverträglichen Katalysatorsystemen oder ermöglichen nur wirtschaftlich unbefriedigende Selektivitäten und Ausbeuten in der Hydroformylierungsstufe zu TCD-Aldehyden. Daher besteht ein Bedarf nach einem möglichst einfachen und kostengünstigen Verfahren zur Hydroformylierung von DCP mit nachfolgender Herstellung von TCD-Alkohol DM.

Die Erfindung besteht daher in einem Verfahren zur Herstellung von 3(4),8(9)-Dihydroxymethyl-tricyclo[5.2.1.0^{2.6}]decan durch Hydroformylierung von Dicyclopentadien mit nachfolgender Hydrierung. Es ist dadurch gekennzeichnet, dass man Dicyclopentadien in einer ersten Hydroformylierungsstufe in einem heterogenen Reaktionssystem unter Verwendung einer wässrigen Lösung, wasserlösliche organische Phosphor(III)verbindungen in komplexer Bindung enthaltender Rhodiumverbindungen bei Rhodium-Konzentrationen von 20 bis 800 Gew.-ppm, bezogen auf die wässrige Katalysatorlösung, und bei Temperaturen von 70 bis 150°C und Drücken von 0,5 bis 10 MPa mit Synthesegas zum 8(9)-Formyl-tricyclo[5.2.1.0^{2.6}]dec-3-en umgesetzt, danach die organische Phase von der wässrigen Phase trennt und anschließend das so erhaltene 8(9)-Formyl-tricyclo[5.2.1.0^{2.6}]dec-3-en in einer zweiten Hydroformylierungsstufe in homogener organischer Phase in Gegenwart von Rhodiumverbindungen bei Rhodium-Konzentrationen von 5 bis 100 Gew.-ppm, bezogen auf das homogene Reaktionsgemisch, oder in Gegenwart von organischen Phosphor(III)-Verbindungen und Rhodiumverbindungen bei Rhodium-Konzentrationen von 5 bis 500 Gew. ppp, bezogen auf das homogene Reaktionsgemisch, und bei Temperaturen von 70 bis 140°C und Drücken von 5 bis 35 MPa durch Umsetzung mit Synthesegas in 3(4),8(9)-Bisformyl-tricyclo[5.2.1.0^{2.6}]decan überführt und das so erhaltene 3(4),8(9)-Bisformyl-tricyclo[5.2.1.0^{2.6}]decan anschließend zum 3(4),8(9)-Dihydroxymethyl-tricyclo[5.2.1.0^{2.6}]decan in Gegenwart von Hydrierkatalysatoren mit Wasserstoff hydriert.

Charakteristisch für das erfindungsgemäße Hydroformylierungsverfahren von Dicyclopentadien ist die zweistufige Reaktionsführung, wobei in der ersten Stufe nach dem heterogenen Zweiphasenprozess in Gegenwart einer wässrigen Katalysatorlösung gearbeitet wird und das Reaktionsprodukt der ersten Stufe, enthaltend überwiegend TCD-Monoaldehyd und geringe Mengen an nicht umgesetzten DCP, ohne weitere Reinigung in einer zweiten Stufe nach Katalysatorzusatz in einem homogenen Reaktionsmedium zum TCD-Dialdehyd umgesetzt wird, das dann anschließend zum TCD-Alkohol DM hydriert wird. Durch diese Art der Reaktionsführung erfolgt in der ersten Reaktionsstufe eine sehr selektive Hydroformylierung der im Sechsring des TCD-Gerüstes vorhandenen Doppelbindung zu TCD-Monoaldehyd, der häufig auch als TCD-Monenal {8(9)-Formyl-tricyclo[5.2.1.0^{2.6}]dec-3-en} bezeichnet wird.

Überraschenderweise zeigt sich, dass das Reaktionsprodukt der ersten Hydroformylierungsstufe nach Abtrennung der wässrigen Katalysatorphase ohne weitere Reinigung in einem homogenen organischen Medium nach Katalysatorzusatz zum TCD-Dialdehyd hydroformyliert werden kann, obwohl die das Wertprodukt enthaltende organische Phase homogen gelöste und analytisch nachweisbare Mengen an Phosphor- und Schwefel-Spalt- und Abbauprodukten enthält, die als Katalysatorgifte in der Oxo-Synthese bekannt sind.

Nach "New Synthesis with Carbon Monoxide" (Edited by J. Falbe, Springer-Verlag 1980, Reactivity and Structure Concepts in Organic Chemisty, Vol. 11, Seite 73) sind bei der rhodiumkatalysierten Hydroformylierung zahlreiche Katalysatorgifte bekannt. Neben Halogen, Acetylenen und Carbonsäuren wird vor allem auch auf Schwefel hingewiesen. Bereits geringe Mengen dieser Katalysatogifte bewirken eine drastische Deaktivierung des Hydroformylierungskatalysators.

Auch für den nachfolgenden Hydrierschritt kann das TCD-Dialdehyd haltige Rohprodukt ohne zwischengeschaltete Reinigungsschritte, wie zum Beispiel Destillations- oder Waschschritte, eingesetzt werden. Die Hydrierung des TCD-Dialdehyds zum TCD-Alkohol DM erfolgt nach konventionellen Verfahren.

Dies ist insofern überraschend, weil auch bei den in der Technik durchgeführten Hydrierverfahren an festbettkatalysatoren der Einfluß von Katalysatorgiften in zahlreichen Publikationen beschrieben wird. In Ullmann's Encyclopedia of Industrial Chemistry, 5. Auflage, 1985, Vol. A1, Seite 283, wird auf die Wirkung von schwefelhaltigen Katalysatorgiften bei heterogenkatalysierten Hydrierverfahren hingewiesen.

Ferner weist die DOS 29 18 107 vor einer Weiterverarbeitung von TCD-Monenal {(8)9-Formyl-tricyclo[5.2.1.0^{2.6}]dec-3-en} zum entsprechenden gesättigten Aldehyd TCD-Monal auf eine destillative Reinigung des ungesättigten Aldehyds hin.

Auch die Lehre in DOS 26 54 221 deutet darauf hin, für den nachfolgenden Hydrierschritt destillativ aufgearbeitetes TCD-Monenal einzusetzen.

Überraschenderweise zeigte sich ebenfalls, dass das in der ersten Stufe nicht vollständig umgesetzte Dicyclopentadien in der zweiten Hydroformylierungsstufe ohne Bildung von hochsiedenden Nebenprodukten vollständig in den TCD-Dialdehyd überführt werden kann. Daraus ergibt sich die vorteilhafte Möglichkeit einer DCP-Teilumsatzfahrweise in der ersten Hydroformylierungsstufe.

Eine destillative Reinigung von TCD-Monenal aus der abgetrennten organischen Phase der ersten Hydroformylierungsstufe ist jedoch nicht ausgeschlossen. Diese Arbeitsweise bedarf jedoch eines zusätzlichen Destillationsschrittes und führt, wenn auch zu nur geringen Destillationsverlusten. Die gezielte Herstellung von TCD-Monenal aus Dicyclopentadien unter Verwendung einer wässrigen Katalysatorlösung sowie die destillative Reinigung ist aus EP-B1-0 186 075 bekannt.

Die erste Reaktionsstufe des neuen Verfahrens führt man als heterogene Reaktion im einem Zweiphasensystem durch, eine Umsetzung, die z.B. in der DE-B-26 27 354 beschrieben ist. Dieser Prozess ist charakterisiert durch das Vorliegen einer organischen Phase, die das olefinische Ausgangsmaterial und das Reaktionsprodukt enthält, und einer wässrigen Phase, in der der Katalysator gelöst ist. Als Katalysatoren werden wasserlösliche Rhodium-Komplexverbindungen eingesetzt, die wasserlösliche organischen Phosphor(III)-Verbindungen als Liganden enthalten. Beispiele für wasserlösliche Phosphor(III)-Verbindungen, die mit Rhodium Komplexverbindungen bilden, sind Triarylphosphine, Trialkylphosphine, gemischte aliphatische-aromatische Phosphine und arylierte bzw. alkylierte Diphosphine, deren organische Reste Sulfonsäuregruppen oder Carboxylgruppen enthalten. Ihre Herstellung und Anwendung ist z.B. aus DE-B 26 27 354, EP-B1-0 103 810, EP-B1-0 163 234 und EP-A1-0 571 819 bekannt. Weitere Gruppen geeigneter Verbindungen sind sulfonierte oder carboxylierte organische Phosphite sowie heterocyclische Verbindungen des dreiwertigen Phosphors, die z.B. aus EP-A1-0 575 785 und EP-A1-0 646 588 bekannt sind.

Als sulfonierte Arylphosphine eignen sich in dem erfindungsgemäßen Verfahren sulfonierte Triarylphosphine der allgemeinen Formel (I) 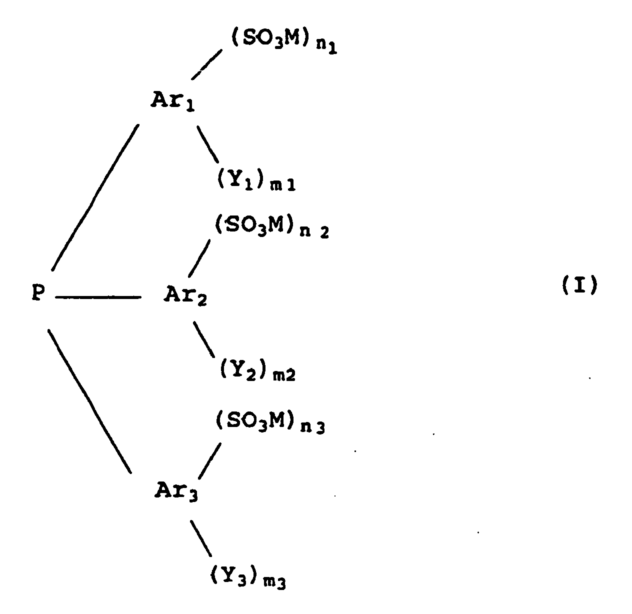 in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR¹R² stehen, in der die Substituenten R¹ und R² gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist.

Zu den Triarylphosphinen zählen bevorzugt solche Triarylphosphine, bei denen die Gruppen Ar¹, Ar², Ar³ Phenylgruppen sind; Y₁, Y₂ und Y₃ für die Methyl-, die Ethylgruppe, für die Methoxy-, Ethoxygruppe und/oder für ein Chloratom stehen; und die kationischen Reste M anorganische Kationen von Natrium, Kalium, Calcium und Barium sind. Insbesondere geeignet sind solche Triarylphosphine, bei denen Ar¹, Ar², Ar³ jeweils eine Phenylgruppe bedeuten, m₁, m₂, m₃ gleich 0 sind, n₁, n₂ und n₃ gleich 0 oder 1 sind und n₁+n₂+n₃ zusammen 1 bis 3 ausmachen, und bei denen die Sulfonat-Gruppen in meta-Stellung stehen.

Ein für die Durchführung des erfindungsgemäßen Hydroformylierungsverfahrens geeignetes Gemisch an (Sulfophenyl)-diphenylphosphin, Di-(sulfophenyl)phenylphosphin und Tri(sulfophenylphosphin) fällt bei der Sulfonierung von Triphenylphosphin an, wie z.B. aus DE-OS 26 27 354 bekannt. Im Stand der Technik wird (Sulfophenyl)diphenylphosphin als TPPMS, Di-(sulfophenyl)phenylphosphin als TPPDS und Tri(sulfophenyl)phosphin als TPPTS abgekürzt.

Als sulfonierte Arylphosphine eignen sich ebenfalls sulfonierte Diphosphine der allgemeinen Formeln (II) oder (III)

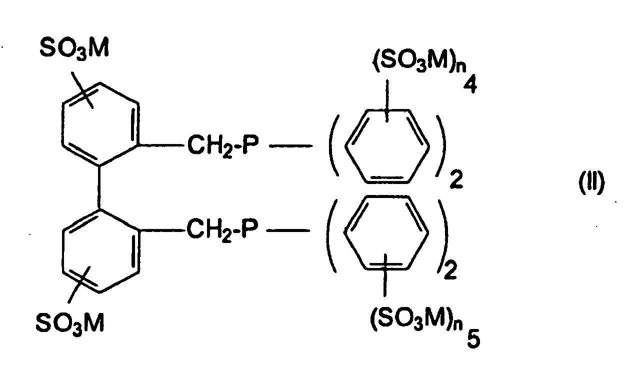

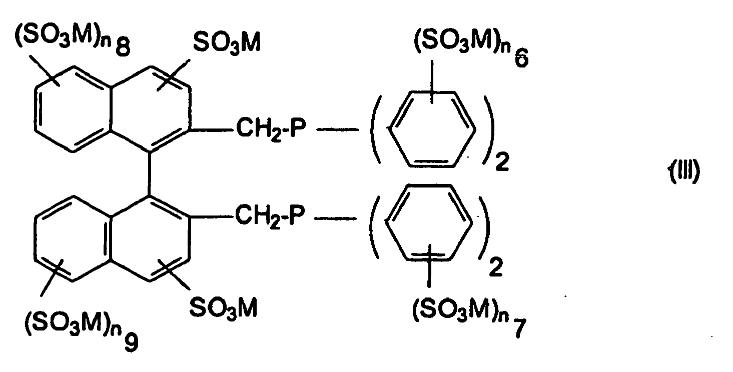

Diese Diphosphine der allgemeinen Formeln (II) und (III) sind aus WO98/30526 bekannt.

In (II) steht ein jedes n₄ und n₅ unabhängig voneinander für 0 oder 1, wobei die Verbindung der Formel (II) bis sechs -SO₃M-Gruppen enthält.

In (III) steht ein jedes n₆, n₇, n₈ und n₉ unabhängig voneinander für 0 oder 1, wobei die Verbindung der Formel (III) vier bis acht -SO₃M-Gruppen enthält.

Aufgrund der Herstellung durch Sulfonierung der entsprechenden Diphosphine der Formeln (IIa) und (IIIa), die keine -SO₃M-Gruppen enthalten, 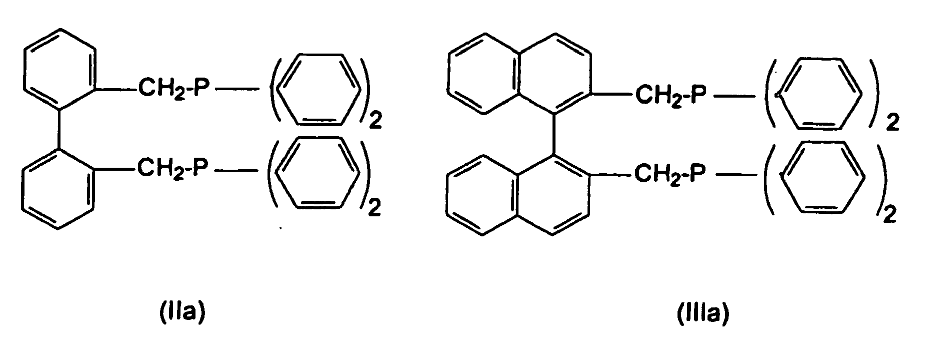 erhält man üblicherweise Gemische von Verbindungen (II) und (III) mit unterschiedlicher Anzahl von -SO₃M-Gruppen. So enthält eine Verbindung der Formeln (II) oder (III), die beispielsweise drei -SO₃M-Gruppen enthält, auch Verbindungen mit lediglich zwei -SO₃M-Gruppen aber auch Verbindungen mit vier oder fünf -SO₃M-Gruppen. Eine Verbindung der Formeln (II) oder (III) mit beispielsweise fünf -SO₃M-Gruppen enthält üblicherweise auch Verbindungen mit lediglich drei oder vier -SO₃M-Gruppen aber auch Verbindungen mit sechs oder sieben -SO₃M-Gruppen.

Verbindungen der Formel (II) besitzen maximal sechs -SO₃M-Gruppen, während Verbindungen der Formel (III) maximal acht -SO₃M-Gruppen aufweisen.

Aus diesem Grund gelangen in der Regel Mischungen von Verbindungen der Verbindungen (II) und (III) mit unterschiedlicher Anzahl von -SO₃M-Gruppen zum Einsatz.

In den Formeln (II) und (III) steht M für Ammonium, ein einwertiges Metall oder das Äquivalent eines mehrwertigen Metalls, insbesondere für Natrium, Kalium, Calcium oder Barium.

Es werden wasserlösliche Komplexverbindungen des Rhodiums eingesetzt.

Die Bedingungen, unter denen die Umsetzung in der ersten Hydroformylierungsstufe abläuft, können innerhalb weiter Grenzen variieren und den individuellen Gegebenheiten angepasst werden. Sie hängen u.a. vom Einsatzmaterial, vom ausgewählten Katalysatorsystem und vom angestrebten Umsetzungsgrad ab. Üblicherweise führt man die Hydroformylierung der Einsatzstoffe bei Temperaturen von 70 bis 150°C durch. Bevorzugt hält man Temperaturen von 100 bis 150°C und insbesondere von 110 bis 140°C ein. Der Gesamtdruck erstreckt sich über einen Bereich von 0,5 bis 10 MPa, vorzugsweise 1 bis 6 MPa und insbesondere 1,5 bis 5 MPa. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Die Rhodium-Konzentration beträgt 20 bis 800 Gew.-ppm, vorzugsweise 50 bis 800 Gew.-ppm und insbesondere 100 bis 600 Gew.-ppm, jeweils bezogen auf die wässrige Katalysatorlösung. Obgleich es möglich ist, als Katalysator die stöchiometrisch zusammengesetzte Rhodium-Phosphor-Komplexverbindung einzusetzen, arbeitet man üblicherweise in Gegenwart von überschüssigem Phosphor-Liganden, d.h. Ligand, der mit Rhodium keine komplexe Bindung eingegangen ist. Je mol Rhodium wendet man bevorzugt 10 bis 300 mol Phosphor in Form einer wasserlöslichen organischen Phosphorverbindung an. Besonders bewährt haben sich molare Verhältnisse von Rhodium zu Phosphor im Bereich von 1:50 bis 1:150. Der Rhodium-Phosphor-Komplexkatalysator braucht nicht einheitlich zusammengesetzt sein, sondern kann z.B. aus einem Gemisch von Rhodium-Komplexverbindungen bestehen, die sich durch die Art der Phosphor-Liganden unterscheiden. Ebenso kann der in der wässrigen Katalysatorlösung enthaltene freie Phosphor-Ligand aus einem Gemisch unterschiedlicher wasserlöslicher organischer Phosphorverbindungen zusammengesetzt sein.

Man bildet den Katalysator üblicherweise aus den Komponenten Rhodium oder Rhodiumverbindung, organische Phosphorverbindung und Synthesegas unter den Bedingungen der Hydroformylierungsreaktion im Reaktionsgemisch. Es ist aber auch möglich, den Katalysator zunächst zu präformieren und ihn anschließend der eigentlichen Hydroformylierungsstufe zuzuführen. Die Bedingungen der Präformierung entsprechen dabei im allgemeinen den Hydroformylierungsbedingungen.

Dicylopentadien kann als solches oder in Lösung der Hydroformylierung zugeführt werden. Geeignete Lösungsmittel sind wasserunlösliche Ketone, Dialkylether, aliphatische Nitrile, aromatische Kohlenwasserstoffe wie Benzol oder Toluol und gesättigte cycloaliphatische Kohlenwasserstoffe wie Cyclopentan oder Cylcohexan oder gesättigte aliphatische Kohlenwasserstoffe.

Um den Umsatz je Zeiteinheit von Dicyclopentadien, das in der wässrigen Katalysatorlösung nur wenig löslich sind, zu erhöhen, kann es sich empfehlen, dieser Lösung ein Phasentransferreagenz (Lösungsvermittler) zuzusetzen. Er verändert die physikalischen Eigenschaften der Grenzflächen zwischen den beiden flüssigen Phasen und erleichtert den Übergang des organischen Reaktanten in die wässrige Katalysatorlösung.

Als Lösungsvermittler sind Verbindungen bekannt, deren hydrophile Gruppen ionisch (anionisch oder kationisch) oder nicht ionisch sind. Zu den anionenaktiven Verbindungen gehören Natrium-, Kalium- oder Ammoniumsalze von Carbonsäuren, vorzugsweise solcher mit 8 bis 20 Kohlenstoffatomen und insbesondere von gesättigten Fettsäuren mit 12 bis 18 Kohlenstoffatomen, ferner Alkylsulfate, Alkylbenzolsulfonate und Alkylbenzolphosphate. Beispiele für kationische Lösungsvermittler sind Tetraalkylammonium- und N-Alkylpyridiniumsalze. Die nichtionischen Phasentransferreagenzien dissoziieren in wässriger Lösung nicht in Ionen. Zu ihnen zählen Alkylpolyethylenglykole, Alkylphenylpolyethylenglykole, Fettsäurealkylolamine und Trialkylaminoxide. Ferner finden Ampholyte wie Aminocarbonsäuren, Betaine und Sulfobetain als Lösungsvermittler Anwendung. Entsprechende Verfahren sind z.B. aus EP-B1-0 157 316 bekannt.

Es können auch Rhodiumkomplexverbindungen eingesetzt werden, die gleichzeitig Katalysator und Phasentransferreagenz sind. Eine solche Arbeitsweise ist z.B. Gegenstand der EP-B1-0 163 234.

Auch hinsichtlich der verfahrenstechnischen und apparativen Ausgestaltung der ersten Stufe des neuen Verfahrens kann man sich innerhalb weiter Grenzen bewegen. Eine bewährte Ausführungsform der heterogenen Hydroformylierung unter Verwendung einer wässrigen Katalysatorphase ist in der EP-B1-0 103 810 beschrieben. Der Reaktionsaustrag der ersten Hydroformylierungsstufe wird in einem Phasentrenner in die organische Produktphase und in die wässrige Katalysatorlösung aufgetrennt. Es hat sich als zweckmäßig erwiesen, die Katalysatorlösung im Kreis zu führen. Die rohe organische Produktphase wird ohne weitere Reinigungsschritte der zweiten Hydroformylierungsstufe zugeführt. Eine zwischengeschaltete destillative Reinigung des Reaktionsproduktes der ersten Hydroformylierungsstufe kann aber gegebenenfalls auch durchgeführt werden.

Die zweite Hydroformylierungsstufe des neuen Verfahrens führt man in einem homogenen Reaktionssystem durch. Der Begriff homogenes Reaktionssystem steht für eine im wesentlichen aus Lösungsmittel, falls in der ersten Stufe und/oder in der zweiten Reaktionsstufe zugesetzt, Katalysator, unumgesetztes Dicyclopentadien und TCD-Monenal zusammengesetzte homogene Lösung. Gegebenenfalls kann sich ein Lösungsmittelzusatz in der zweiten Reaktionsstufe als zweckmäßig erweisen. Als Lösungsmittel werden organische Verbindungen eingesetzt, in denen Ausgangsmaterial, Reaktionsprodukt und Katalysatorsystem löslich sind. Beispiele für solche Verbindungen sind aromatische Kohlenwasserstoffe, wie Benzol und Toluol oder die isomeren Xylole und Mesitylen. Andere gebräuchliche Lösungsmittel sind Paraffinöl, Cyclohexan, n-Hexan, n-Heptan oder n-Octan, Ether, wie Tetrahydrofuran, Ketone oder Texanol® der Firma Eastman. Der Anteil des Lösungsmittels im Reaktionsmedium kann über einen weiten Bereich variiert werden und beträgt üblicherweise zwischen 10 und 80 Gew.-%, vorzugsweise 20 bis 50 Gew.-%, bezogen auf das Reaktionsgemisch.

Ein Lösungsmittelzusatz in der zweiten, wie auch in der ersten Hydroformylierungsstufe, ist jedoch nicht zwingend erforderlich.

Als Katalysatoren in der zweiten Hydroformylierungsstufe verwendet man Rhodiumverbindungen. Dabei werden Rhodium-Verbindungen verwendet, die nicht mit phosphorhaltigen Liganden, wie Phosphinen oder Phosphiten modifiziert sind. Solche, nicht mit Phosphinen oder Phosphiten modifizierten Rhodiumkatalysatoren und ihre Eignung als Katalysator zur Hydroformylierung sind aus der Literatur bekannt und sie werden als unmodifizierte Rhodiumkatalysatoren bezeichnet. Es wird in der Fachliteratur angenommen, dass die Rhodium-Verbindung HRh(CO)₄ die katalytisch aktive Rhodiumspezies bei der Hydroformylierung mit unmodifizierten Rhodiumkatalysatoren ist, obgleich dies aufgrund der vielen in der Hydroformylierungszone nebeneinander ablaufenden Chemismen nicht eindeutig bewiesen ist. Da im allgemeinen die Verwendung von nicht mit Phosphinen modifizierten Rhodiumkatalysatoren einen geringeren Rhodiumgehalt erfordert, arbeitet man in der zweiten Hydroformylierungsstufe mit unmodifizierten Rhodiumkatalysatoren. Der Rhodiumgehalt beträgt 5 bis 100 ppm, bezogen auf das homogene Reaktionsgemisch.

Man kann aber auch in der zweiten Hydroformylierungsstufe Rhodium-Komplexverbindungen verwenden, die organische Phosphor(III)-Verbindungen als Liganden enthalten. Derartige Komplexverbindungen und ihre Herstellung sind bekannt (z.B. aus US-A-3 527 809, US-A-4 148 830, US-A-4 247 486, US-A-4 283 562). Sie können als einheitliche Komplexverbindungen oder auch als Gemisch unterschiedlicher Komplexverbindungen eingesetzt werden. Die Rhodium-Konzentration im Reaktionsmedium erstreckt sich über einen Bereich von 5 bis etwa 500 Gew.-ppm und beträgt vorzugsweise 10 bis 500 Gew.-ppm. Insbesondere wendet man Rhodium in Konzentrationen von 20 bis 500 Gew.-ppm, jeweils bezogen auf das homogene Reaktionsgemisch an. Als Katalysator kann die stöchiometrisch zusammengesetzte Rhodium-Komplexverbindung Anwendung finden. Es hat sich jedoch als zweckmäßig erwiesen, die Hydroformylierung in Gegenwart eines Katalysatorsystems aus Rhodium-Phosphor-Komplexverbindung und freiem, d.h. überschüssigen Phosphor-Liganden durchzuführen, der mit Rhodium keine Komplexverbindung mehr eingeht. Der freie Phosphor-Ligand kann der gleiche sein, wie in der Rhodium-Komplexverbindung, es können aber auch von diesem verschiedene Liganden eingesetzt werden. Der freie Ligand kann eine einheitliche Verbindung sein oder aus einem Gemisch verschiedener Organophosphorverbindungen bestehen. Beispiele für Rhodium-Phosphor-Komplexverbindungen, die als Katalysatoren Anwendung finden können, sind in US-A-3 527 809 beschrieben. Zu den bevorzugten Liganden in den Rhodium-Komplexkatalysatoren zählen z. B. Triarylphosphine wie Triphenylphosphin, Trialkylphosphine wie Tri-(n-octyl)-phosphin, Trilaurylphosphin, Tri-(cyclohexyl)-phosphin, Alkylphenylphosphine, Cycloalkylphenylphosphine und organische Diphosphite. Wegen seiner leichten Zugänglichkeit wird Triphenylphosphin besonders häufig angewandt.

Arbeitet man mit einem modifizierten Rhodium-Komplexkatalysatorsystem, beträgt üblicherweise in der homogenen Reaktionsmischung das molare Verhältnis von Rhodium zu Phosphor 1 : 5 bis 1 : 200, jedoch kann der molare Anteil des Phosphors in Form organischer Phosphorverbindungen auch höher sein. Vorzugsweise setzt man Rhodium und organisch gebundenen Phosphor in molaren Verhältnissen von 1 : 10 bis 1 : 100 ein.

Die Bedingungen, unter denen die Umsetzung in der zweiten Hydroformylierungsstufe abläuft, können innerhalb weiter Grenzen variieren und den individuellen Gegebenheiten angepasst werden. Sie hängen u.a. vom Einsatzmaterial, vom ausgewählten Katalysatorsystem und vom angestrebten Umsetzungsgrad ab. Üblicherweise führt man die zweite Hydroformylierungsstufe des rohen TCD-Monenal's bei Temperaturen von 70 bis 140°C durch. Bevorzugt hält man Temperaturen von 80 bis 130°C und insbesondere von 90 bis 120°C ein. Der Gesamtdruck erstreckt sich über einen Bereich von 5 bis 35 MPa, vorzugsweise 10 bis 30 MPa und insbesondere 20 bis 30 MPa. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Man bildet den Katalysator üblicherweise aus den Komponenten Rhodium oder Rhodiumverbindung und Synthesegas unter den Bedingungen der Hydroformylierungsreaktion im Reaktionsgemisch, gegebenenfalls in Gegenwart von organischen Phosphor(III)-Verbindungen. Es ist aber auch möglich, den Katalysator zunächst zu präformieren und ihn anschließend der eigentlichen Hydroformylierungsstufe zuzuführen. Die Bedingungen der Präformierung entsprechen dabei im allgemeinen den Hydroformylierungsbedingungen.

Zur Herstellung des Hydroformylierungskatalysators für die erste und zweite Reaktionsstufe gelangt Rhodium, entweder in metallischer Form oder als Verbindung zum Einsatz. In metallischer Form verwendet man Rhodium entweder als feinverteilte Partikel oder in dünner Schicht auf einem Träger, wie Aktivkohle, Calciumcarbonat, Aluminiumsilikat, Tonerde niedergeschlagen. Als Rhodiumverbindungen eignen sich Salze aliphatischer Mono- und Polycarbonsäuren, wie Rhodium-2-Ethylhexanoat, -Acetat, -Oxalat, -Propionat oder -Malonat. Weiterhin können Salze anorganischer Wasserstoff- und Sauerstoffsäuren, wie Nitrate oder Sulfate, die verschiedenen Rhodiumoxide oder auch Rhodiumcarbonylverbindungen, wie Rh₃(CO)₁₂, Rh₆(CO)₁₆, Rhodium-Komplexverbindungen, z.B. Cyclopentadienylrhodiumverbindungen, Rhodiumacetylacetonat, oder [RhCl(Cyclooctadien-1,5]₂ eingesetzt werden. Rhodiumhalogenverbindungen kommen wegen ihres korrosiven Verhaltens der Halogenidionen weniger in Betracht.

Bevorzugt werden Rhodiumoxide und insbesondere Rhodiumacetat und -2-ethylhexanoat.

Die einzelnen Hydroformylierungsstufen können sowohl absatzweise als auch kontinuierlich durchgeführt werden.

Das Umsetzungsprodukt der zweiten Hydroformylierungsstufe wird ohne weitere Reinigung und ohne Katalysatorabtrennung der Hydrierstufe zugeführt.

Die Hydrierung des rohen TCD-Dialdehyds zum TCD-Alkohol DM erfolgt unter allgemein üblichen Reaktionsbedingungen in Gegenwart konventioneller Hydrierkatalysatoren. Im allgemeinen beträgt die Hydriertemperatur 70 bis 170°C und der angewandte Druck 1 bis 30 MPa. Als Hydrierkatalysatoren sind besonders Nickelkatalysatoren geeignet.

Das katalytisch aktive Metall kann auf einem Träger aufgebracht werden, im allgemeinen in einer Menge von etwa 5 bis etwa 70 Gew.-%, vorzugsweise etwa 10 bis etwa 65 Gew.-% und insbesondere etwa 20 bis etwa 60 Gew.-% jeweils bezogen auf das Gesamtgewicht des Katalysators. Als Katalysatorträger eignen sich alle herkömmlichen Trägermaterialien, z.B. Aluminiumoxid, Aluminiumoxidhydrate in ihren verschiedenen Erscheinungsformen, Siliciumdioxid, Polykieselsäuren (Kieselgele) einschließlich Kieselgur, Kieselxerogele, Magnesiumoxid, Zinkoxid, Zirconiumoxid und Aktivkohle. Neben den Hauptkomponenten Nickel und Trägermaterial können die Katalysatoren noch Zusatzstoffe in untergeordneten Mengen enthalten, die z.B. der Verbesserung ihrer Hydrieraktivität und/oder ihrer Standzeit und/oder ihrer Selektivität dienen. Derartige Zusatzstoffe sind bekannt, zu ihnen gehören z.B. die Oxide des Natriums, Kaliums, Magnesiums, Calciums, Bariums, Zinks, Aluminiums, Zirconiums und Chroms. Sie werden dem Katalysator im allgemeinen in einem Anteil von insgesamt 0,1 bis 50 Gew.-Teile bezogen auf 100 Gew.-Teile Nickel zugesetzt.

Aber auch trägerfreie Katalysatoren, wie Raney-Nickel oder Raney-Kobalt können in dem Hydrierprozess verwendet werden.

Die Hydrierstufe wird diskontinuierlich oder kontinuierlich in flüssiger Phase mit suspendierten Katalysatoren oder in flüssiger oder gasförmiger Phase mit fest angeordneten Katalysatoren durchgeführt; die kontinuierliche Arbeitsweise wird bevorzugt.

Bei diskontinuierlicher Verfahrensführung verwendet man, bezogen auf TCD-Dialdehyd, 1 bis 10, vorzugsweise 2 bis 6 Gew.-% Nickel in Form der vorstehend beschriebenen Katalysatoren. Bei kontinuierlicher Arbeitsweise setzt man je Liter Katalysator und Stunde etwa 0,05 bis etwa 5,0 kg des TCD-Dialdehyds ein, bevorzugt werden etwa 0,1 bis 2,0 kg TCD-Dialdehyd je Liter Katalysator und Stunde.

Die Hydrierung erfolgt vorzugsweise mit reinem Wasserstoff. Es können jedoch auch Gemische verwendet werden, die freien Wasserstoff und daneben unter den Hydrierbedingungen inerte Bestandteile enthalten. In jedem Fall ist dafür Sorge zu tragen, dass das Hydriergas frei von Katalysatorgiften wie Schwefelverbindungen oder Kohlenmonoxid in schädlichen Mengen ist.

Überraschenderweise wirken sich die im rohen TCD-Alkohol DM vorhandenen phosphor- und schwefelhaltigen Spalt- und Abbauprodukte nicht schädlich auf die Hydrieraktivität des Katalysators aus. Rhodium aus dem nicht abgetrennten Hydroformylierungskatalysator der zweiten Hydroformylierungsstufe schlägt sich nahezu vollständig auf dem Hydrierkatalysator nieder. Es kann nach bekannten Verfahren zurückgewonnen werden.

Roher TCD-Dialdehyd kann als solcher oder zusammen mit einem Lösungs- oder Verdünnungsmittel eingesetzt werden, wobei die zuerst genannte Variante bevorzugt wird. Setzt man ein Lösungs- oder Verdünnungsmittel hinzu, ist die Auswahl der Lösungs- oder Verdünnungsmittel, die reine Substanzen aber auch Substanzgemische sein können, nicht kritisch sofern sichergestellt ist, dass sie mit dem Einsatzstoff eine homogene Lösung bilden. Beispiele für geeignete Lösungs- oder Verdünnungsmittel sind lineare oder cyclische Ether wie Tetrahydrofuran oder Dioxan. Die Menge des eingesetzten Lösungs- oder Verdünnungsmittels kann entsprechend den apparativen und verfahrenstechnischen Gegebenheiten frei gewählt werden, im allgemeinen setzt man Lösungen ein, die 10 bis 75 Gew.-% TCD-Dialdehyd enthalten. Besonders bewährt hat es sich im Rahmen des erfindungsgemäßen Verfahrens, den bei der Hydrierung entstandenen TCD-Alkohol DM als Lösungs- oder Verdünnungsmittel zu verwenden. In diesem Fall wird, bezogen auf das Gewicht des TCD-Dialdehyds, zweckmäßig die 1- bis 30-fache, vorzugsweise die 5- bis 20-fache und insbesondere die 5- bis 10-fache Menge des TCD-Alkohol DM als Lösungs- und Verdünnungsmittel zugesetzt.

Die Gewinnung des reinen TCD-Alkohol DM erfolgt nach konventionellen Destillationsverfahren. TCD-Alkohol DM wird als Kopfprodukt abgezogen. Restmengen des in der zweiten Hydroformylierungsstufe eingesetzten Rhodiums fallen im Destillationsrückstand an und werden nach bekannten Verfahren zurückgewonnen.

Im Folgenden wird das erfindungsgemäße Verfahren anhand einiger Beispiel näher erläutert, es ist jedoch nicht auf die beschriebenen Ausführungsformen beschränkt.

### Beispiele

Die bei der analytischen Charakterisierung der Reaktionsprodukte verwendeten Abkürzungen haben folgende Bedeutung:

| | |
|---|---|
| DCP | Dicyclopentadien |
| TCD-Monenal | 8(9)-Formyl-tricyclo[5.2.1.0^{2.6}]dec-3-en |
| TCD-Dial | 3(4),8(9)-Bisformyl-tricyclo[5.2.10^{2.6}]decan |
| Tri-CP | Tricyclopentadien. |
| TPPTS bedeutet | Natrium-Triphenylphosphintrisulfonat |

### Herstellung von TCD-Alkohol DM

### 1. Herstellung von TCD-Monenal

In einem 5 I-Autoklaven werden 2.119 g TPPTS-Lösung mit einem P(III)-Gehalt von 472 mmol/kg vorgelegt und mit 160,2 g Rh-Lösung (Rh-Gehalt: 6.423 mg/kg) versetzt. Danach wird eine Mischung aus 661,1 g Dicyclopentadien (technisch, DCP-Gehalt: 93,72 Gew.-%) und 283,0 g Toluol zugegeben. Das Reaktionsgemisch wird auf 135°C erwärmt und bei einem Synthesegasdruck von 2,5 MPa und einer Reaktionszeit von 6 Stunden umgesetzt.

Nach Reaktionsende wird abgekühlt und die obere, organische Phase von der wässrigen Katalysatorphase durch Phasentrennung abgetrennt. Die verbleibende Katalysatorphase wird erneut mit einem Gemisch aus Dicyclopentadien und Toluol versetzt und erneut umgesetzt. Dieser Vorgang wird insgesamt achtmal wiederholt.

Die organischen Phasen (Summe: 9.923 g) werden vereinigt und gaschromatographisch untersucht.

### GC-Analyse (in Fl.-%, Flächenprozent)

| | |
|---|---|
| Vortaufkomponenten | 0,32 |
| Toluol | 29,45 |
| DCP | 4,55 |
| TCD-Monenal | 61,30 |
| TCD-Dial | 0,81 |
| Tri-CP | 0,42 |
| Sonstige | 3,15 |

### 2. Herstellung von TCD-Dialdehyd

400 g rohes TCD-Monenal aus der ersten Reaktionsstufe werden ohne Anwendung weiterer Reinigungsschritte durch Zugabe einer toluolischen Lösung von Rh-2-ethylhexanoat auf einen Rhodiumgehalt von 20 ppm, bezogen auf die gesamte Reaktionslösung, eingestellt und in einem 1-I Autoklav vorgelegt. Das Reaktionsgemisch wird auf 120°C erwärmt und bei einem Druck von 26,0 MPa und einer Reaktionszeit von 6 Stunden umgesetzt. Nach Reaktionsende wird abgekühlt und entspannt, das erhaltene Reaktionsprodukt (455,9 g) wird gaschromatographisch untersucht.

### GC-Analyse (in Fl.-%)

| | |
|---|---|
| Vorlaufkomponenten | 1,30 |
| Toluol | 31,70 |
| TCD-Monenal | 2,32 |
| TCD-Dial | 62,36 |
| Sonstige | 2.32 |

### 3. Herstellung von TCD-Alkohol-DM

Der nach der zweiten Hydroformylierungsstufe erhaltene TCD-Dialdehyd wird ohne weitere Reinigung in die Hydrierung eingesetzt. Hierzu werden 450 g TCD-Dialdehyd aus der zweiten Stufe und 40 g Ni 52/35 - Katalysator der Firma Johnson-Matthey Plc - in einem 1-I Autoklav vorgelegt. Das Reaktionsgemisch wird auf 120°C erwärmt und bei einem Druck von 10,0 MPa und einer Reaktionszeit von 8 Stunden umgesetzt. Nach Reaktionsende wird abgekühlt, entspannt und vom Katalysator abfiltriert. Das so erhaltene Reaktionsprodukt (456,0 g) wird gaschromatographisch untersucht.

### GC-Analyse (in Fl.-%)

| | |
|---|---|
| Vorlaufkomponenten | 1,37 |
| Toluol / Methylcyclohexan | 30,20 |
| TCD-Alkohol M | 2,52 |
| TCD-Alkohol DM | 63,57 |
| Sonstige | 2,34 |

Zur Aufarbeitung wird das rohe Hydrierprodukt (450,0 g) an einer Claisenbrücke destilliert. Man erhält 301,6 g Hauptfraktion in einem Siedebereich von 148 - 210°C bei einem Druck von 1 hPa mit folgender Zusammensetzung:

### GC-Analyse (in Fl.-%)

| | |
|---|---|
| Vorlaufkomponenten | 0,15 |
| TCD-Alkohol M | 3,46 |
| TCD-Alköhol DM | 96,13 |
| Sonstige | 0,26 |

Die Gesamtausbeute an TCD-Alkohol DM über alle Stufen beträgt 89,3 % d.Th., bezogen auf eingesetztes Dicyclopentadien. TCD-Alkohol M bedeutet den monofunktionellen Alkohol 8(9)-Hydroxymethyl-tricyclo[5.2.1.0^{2.6}]decan.

Das erfindungsgemäße Verfahren ermöglicht die Herstellung von TCD-Alkohol DM in hohen Ausbeuten, wobei auf die aufwendige Reinigung der Zwischenstufen verzichtet werden kann.

## Patentansprüche

1. Verfahren zur Herstellung von 3(4),8(9)-Dihydroxymethyltricyclo[5.2.1.0^{2.6]}decan durch Hydroformylierung von Dicyclopentadien mit nachfolgender Hydrierung, **dadurch gekennzeichnet, dass** man Dicyclopentadien in einer ersten Hydrofomylierungsstufe in einem heterogenen Reaktionssystem unter Verwendung einer wässrigen Lösung, wasserlösliche organische Phosphor(III)verbindungen in komplexer Bindung enthaltender Rhodiumverbindungen bei Rhodium-Konzentrationen von 20 bis 800 Gew.-ppm, bezogen auf die wässrige Katalysatorlösung, und bei Temperaturen von 70 bis 150°C und Drücken von 0,5 bis 10 MPa mit Synthesegas zum 8(9)-Formyl-tricyclo[5.2.1.0^{2.6}]dec-3-en umgesetzt, danach die organische Phase von der wässrigen Phase trennt und anschließend das so erhaltene 8(9)-Formyl-tricyclo[5.2.1.0^{2.6}]dec-3-en in einer zweiten Hydroformylierungsstufe in homogener organischer Phase in Gegenwart von Rhodiumverbindungen bei Rhodium-Konzentrationen von 5 bis 100 Gew.-ppm, bezogen auf das homogene Reaktionsgemisch, oder in Gegenwart von organischen Phosphor(III)-Verbindungen und Rhodiumverbindungen bei Rhodium-Konzentrationen von 5 bis 500 Gew. ppm, bezogen auf das homogene Reaktionsgemisch, und bei Temperaturen von 70 bis 140°C und Drücken von 5 bis 35 MPa durch Umsetzung mit Synthesegas in 3(4),8(9)-Bisformyltricyclo[5.2.1.0^{2.6}]decan überführt und das so erhaltene 3(4),8(9)-Bis-formyl-tricyclo[5.2.1.0^{2.6}]decan anschließend zum 3(4),8(9)-Dihydroxymethyl-tricyclo[5.2.1.0^{2.6}]decan in Gegenwart von Hydrierkatalysatoren mit Wasserstoff hydriert.

2. Verfahren gemäß Anspruch 1, **dadurch gekennzeichnet, dass** das in der ersten Hydroformylierungsstufe erhaltene 8(9)-Formyl-tricyclo[5.2.1.0^{2.6}]dec-3-en vor dem Einsatz in die zweite Hydroformylierungsstufe destilliert wird.

3. Verfahren gemäß Anspruch 1, **dadurch gekennzeichnet, dass** man als organische Phosphor(III)-Verbindungen Triarylphosphine, Trialkylphosphine, Alkylphenylphosphine, Cycloalkylphenylphosphine und organische Diphosphite verwendet.

4. Verfahren gemäß einem oder mehreren der Ansprüch 1 bis 3, **dadurch gekennzeichnet, dass** in der ersten Hydroformylierungsstufe als wasserlösliche organische Phoshor(III)-Verbindungen sulfonierte Triarylphosphine der allgemeinen Formel (I) 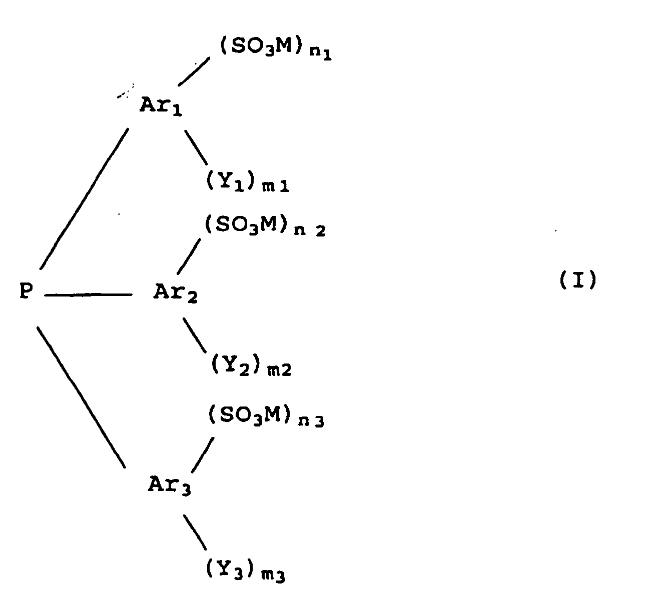 eingesetzt werden, in der Ar¹, Ar² und Ar³ gleiche oder verschiedene Arylgruppen mit 6 bis 14 Kohlenstoffatomen bedeuten, die Substituenten Y₁, Y₂ und Y₃ gleiche oder verschiedene geradkettige oder verzweigte Alkyl- oder Alkoxyreste mit 1 bis 4 Kohlenstoffatomen, Chlor, Brom, die Hydroxyl-, Cyanid- oder Nitrogruppe bedeuten, ferner für die Aminogruppe der Formel NR¹R² stehen, in der die Substituenten R¹ und R² gleich oder verschieden sind und Wasserstoff, geradkettige oder verzweigte Alkylgruppen mit 1 bis 4 Kohlenstoffatomen bedeuten, in der M für Lithium, Natrium, Kalium, Magnesium, Calcium oder Barium steht, in der m₁, m₂ und m₃ gleich oder verschieden sind und ganze Zahlen von 0 bis 5 bedeuten, in der n₁, n₂ und n₃ gleich oder verschieden sind und für ganze Zahlen von 0 bis 3 stehen, wobei mindestens eine der Zahlen n₁, n₂ und n₃ gleich oder größer 1 ist.

5. Verfahren gemäß Anspruch 4, **dadurch gekennzeichnet, dass** Ar₁, Ar₂, Ar₃ jeweils eine Phenylgruppe bedeuten, m₁, m₂, m₃ gleich 0 sind, n₁, n₂, n₃ gleich 0 oder 1 sind und n₁+n₂+n₃ zusammen 1 bis 3 ausmachen, und dass die Sulfonat-Gruppen in meta-Stellung stehen.

6. Verfahren gemäß einem oder mehreren der Ansprüche 1 bis 3, **dadurch gekennzeichnet, dass** in der ersten Hydroformylierungsstufe als wasserlösliche organische Phosphor(III)-Verbindungen sulfonierte Diphosphine der allgemeinen Formel (II) 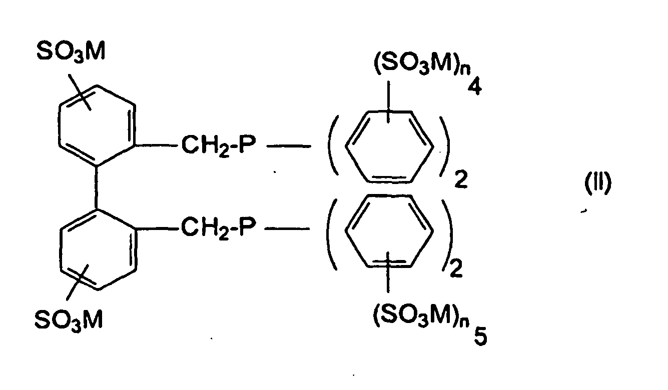 in der n₄ und n₅ unabhängig voneinander für 0 oder 1 steht, wobei die sulfonierten Diphosphine der allgemeinen Formel (II) bis zu sechs SO₃M-Gruppen enthalten, und M Ammonium, ein einwertiges Metall oder das Äquivalent eines mehrwertigen Metalls bedeutet.

7. Verfahren gemäß einem oder mehreren der Ansprüche 1 bis 3, **dadurch gekennzeichnet, dass** in der ersten Hydroformylierungsstufe als wasserlösliche organische Phosphor(III)-Verbindungen sulfonierte Diphosphine der allgemeinen Formel (III) 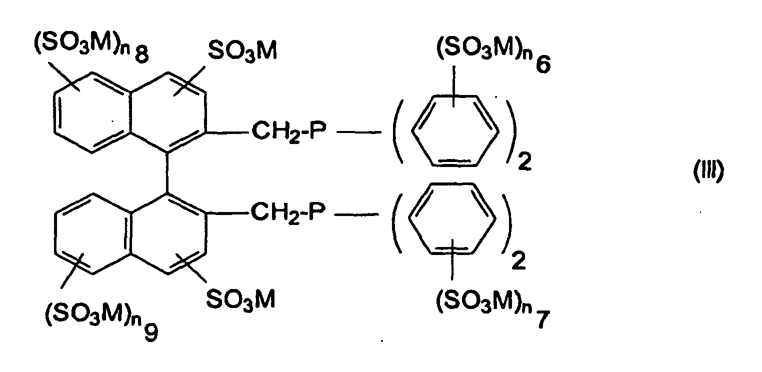 in der n_{6,} n_{7,} n₈ und n₉ unabhängig voneinander für 0 oder 1 steht, wobei die sulfonierten Diphosphine der allgemeinen Formel (III) vier bis acht SO₃M-Gruppen enthalten, und M Ammonium, ein einwertiges Metall oder das Äquivalent eines mehrwertigen Metalls bedeutet.

8. Verfahren gemäß einem oder mehreren der Ansprüche 1 bis 7, **dadurch gekennzeichnet, dass** in der ersten Hydroformylierungsstufe die Temperatur 100 bis 150°C, bevorzugt 110 bis 140°C, und der Druck 1 bis 6 MPa, bevorzugt 1,5 bis 5 MPa, beträgt.

9. Verfahren gemäß einem oder mehreren der Ansprüche 1 bis 8, **dadurch gekennzeichnet, dass** in der zweiten Hydroformylierungsstufe die Temperatur 80 bis 130°C, bevorzugt 90 bis 120°C, und der Druck 10 bis 30 MPa, bevorzugt 20 bis 30 MPa beträgt.

10. Verfahren gemäß einem oder mehreren der Ansprüche 1 bis 9, **dadurch gekennzeichnet, dass** in der ersten Hydroformylierungsstufe die Rhodium-Konzentration 50 bis 800 Gew.-ppm und insbesondere 100 bis 600 Gew.-ppm, jeweils bezogen auf die wässrige Katalysatorlösung, beträgt.

11. Verfahren gemäß einem oder mehreren der Ansprüche 1 bis 10, **dadurch gekennzeichnet, dass** in der ersten Hydroformylierungsstufe je mol Rhodium 10 bis 300 mol, insbesondere 50 bis 150 mol, Phosphor in Form der wasserlöslichen organischen Phosphorverbindung verwendet werden.

12. Verfahren gemäß einem oder mehreren der Ansprüche 1 bis 11, **dadurch gekennzeichnet, dass** in der zweiten Hydroformylierungsstufe in Gegenwart von organischen Phosphor(III)-Verbindungen die Rhodium-Konzentration 10 bis 500 Gew.-ppm und insbesondere 20 bis 500 Gew.-ppm, jeweils bezogen auf das homogene Reaktionsgemisch, beträgt und je mol Rhodium 5 bis 200 mol, vorzugsweise 10 bis 100 mol Phosphor in Form organischer Phosphorverbindungen verwendet werden.

## Claims

1. A process for preparing 3(4),8(9)-dihydroxymethyltricyclo[5.2.1.0^{2,6}]decane by hydroformylating dicyclopentadiene with subsequent hydrogenation, which comprises reacting dicyclopentadiene, in a first hydroformylation stage in a heterogeneous reaction system using an aqueous solution of rhodium compounds, containing water-soluble organic phosphorus(III) compounds in complex-bound form, at rhodium concentrations of from 20 to 800ppm by weight, based on the aqueous catalyst solution, and at temperatures of from 70 to 150°C and pressures of from 0.5 to 10 MPa, with synthesis gas to give 8(9)-formyltricyclo[5.2.1.0²'⁶]dec-3-ene, then separating the organic phase from the aqueous phase and subsequently converting the thus obtained 8(9)-formyltricyclo[5.2.1.0^{2,6}] dec-3-ene, in a second hydroformylation stage in homogeneous organic phase in the presence of rhodium compounds at rhodium concentrations of from 5 to 100 ppm by weight, based on the homogeneous reaction mixture, or in the presence of organic phosphorus(III) compounds and rhodium compounds at rhodium concentrations of from 5 to 500 ppm by weight, based on the homogeneous reaction mixture, and at temperatures of from 70 to 140°C and pressures of from 5 to 35 MPa by reacting with synthesis gas, to 3(4),8(9)-bisformyltricyclo[5.2.1.0^{2,6}]decane and subsequently hydrogenating the thus obtained 3(4),8(9)-bisformyltricyclo[5.2.1.0^{2,6}]decane with hydrogen in the presence of hydrogenation catalysts to give 3(4),8(9)-dihydroxymethyltricyclo[5.2.1.0^{2,6}]decane.

2. The process as claimed in claim 1, wherein the 8(9)-formyltricyclo[5.2.1.0^{2,6}] dec-3-ene obtained in the first hydroformylation stage is distilled before use in the second hydroformylation stage.

3. The process as claimed in claim 1, wherein the organic phosphorus(III) compounds used are triarylphosphines, trialkylphosphines, alkylphenylphosphines, cycloalkylphenylphosphines and organic diphosphites.

4. The process as claimed in one or more of claims 1 to 3, wherein the water-soluble organic phosphorus(III) compounds used in the first hydroformylation stage are sulfonated triarylphosphines of the formula (I) 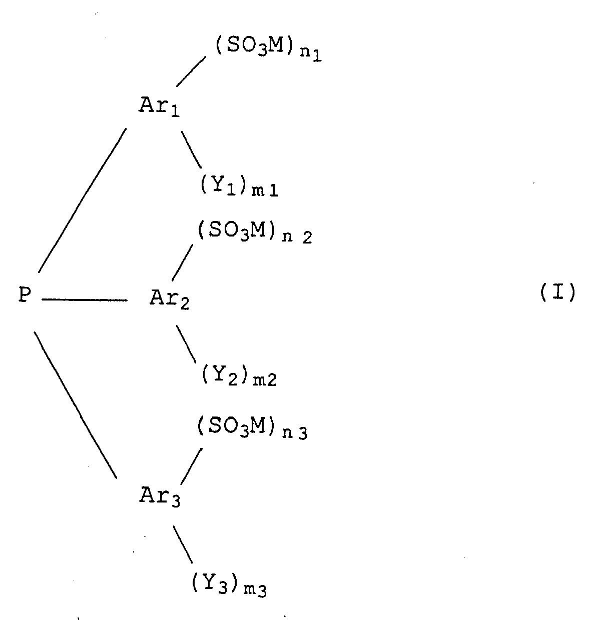 in which Ar¹, Ar² and Ar³ are identical or different aryl groups having from 6 to 14 carbon atoms, the substituents Y₁, Y₂ and Y₃ are identical or different, straight-chain or branched alkyl or alkoxy radicals having from 1 to 4 carbon atoms, chlorine, bromine, the hydroxyl, cyanide or nitro group, and also the amino group of the formula NR¹R² in which the substituents R¹ and R² are the same or different and are each hydrogen, straight-chain or branched alkyl groups having from 1 to 4 carbon atoms, in which M is lithium, sodium, potassium, magnesium, calcium or barium, in which m₁, m₂ and m₃ are the same or different and are each integers from 0 to 5, in which n₁, n₂ and n₃ are the same or different and are each integers from 0 to 3, and at least one of the numbers n₁, n₂ and n₃ is equal to or greater than 1.

5. The process as claimed in claim 4, wherein Ar₁, Ar₂, Ar₃ are each a phenyl group, m₁, m₂, m₃ are each 0, n₁, n₂, n₃ are each 0 or 1 and n₁+n₂+n₃ together add up to from 1 to 3, and the sulfonate groups are in the meta-position.

6. The process as claimed in one or more of claims 1 to 3, wherein the water-soluble organic phosphorus(III) compounds used in the first hydroformylation stage are sulfonated diphosphines of the formula (II) 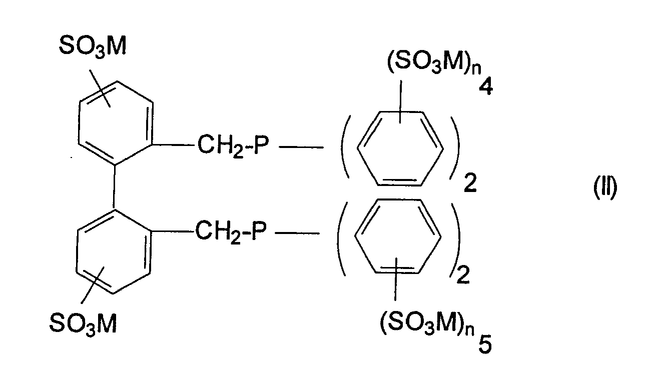 in which n₄ and n₅ are each independently 0 or 1, and the sulfonated diphosphines of the formula (II) contain up to six SO₃M groups, and M is ammonium, a monovalent metal or the equivalent of a polyvalent metal.

7. The process as claimed in one or more of claims 1 to 3, wherein the water-soluble organic phosphorus (III) compounds used in the first hydroformylation stage are sulfonated diphosphines of the formula (III) 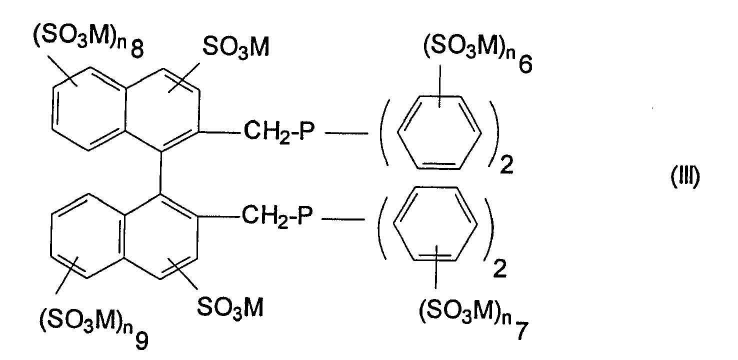 in which n₆, n₇, n₈ and n₉ are each independently 0 or 1, and the sulfonated diphosphines of the formula (III) contain from four to eight SO₃M groups, and M is ammonium, a monovalent metal or the equivalent of a polyvalent metal.

8. The process as claimed in one or more of claims 1 to 7, wherein the temperature in the first hydroformylation stage is from 100 to 150°C, preferably from 110 to 140°C, and the pressure is from 1 to 6 MPa, preferably from 1.5 to 5 MPa.

9. The process as claimed in one or more of claims 1 to 8, wherein the temperature in the second hydroformylation stage is from 80 to 130°C, preferably from 90 to 120°C, and the pressure is from 10 to 30 MPa, preferably from 20 to 30 MPa.

10. The process as claimed in one or more of claims 1 to 9, wherein the rhodium concentration in the first hydroformylation stage is from 50 to 800 ppm by weight and especially from 100 to 600 ppm by weight, based in each case on the aqueous catalyst solution.

11. The process as claimed in one or more of claims 1 to 10, wherein from 10 to 300 mol, especially from 50 to 150 mol, of phosphorus in the form of the water-soluble organic phosphorus compound are used per mole of rhodium in the first hydroformylation stage.

12. The process as claimed in one or more of claims 1 to 11, wherein the rhodium concentration in the second hydroformylation stage in the presence of organic phosphorus(III) compounds is from 10 to 500 ppm by weight and especially from 20 to 500 ppm by weight, based in each case on the homogeneous reaction mixture, and from 5 to 200 mol, preferably from 10 to 100 mol, of phosphorus in the form of organic phosphorus compounds are used per mole of rhodium.

## Revendications

1. Procédé pour la préparation de 3(4),8(9)-dihydroxyméthyltricyclo[5.2.1.0^{2.6}]décane par hydroformylation de dicyclopentadiène avec une hydrogénation consécutive, **caractérisé en ce qu'**on transforme du dicyclopentadiène dans une première étape d'hydroformylation dans un système de réaction hétérogène, en utilisant une solution aqueuse de composés du rhodium contenant, en liaison complexe, des composés organiques solubles dans l'eau du phosphore (III), à des concentrations en rhodium de 20 à 800 ppm en poids, par rapport à la solution aqueuse de catalyseur et à des températures de 70 à 150°C et des pressions de 0,5 à 10 MPa avec du gaz de synthèse en 8(9)-formyltricyclo[5.2.1.0^{2.6}]déc-3-ène, puis on sépare la phase organique de la phase aqueuse et on transforme ensuite le 8(9)-formyltricyclo[5.2.1.0^{2.6}]déc-3-ène dans une deuxième étape d'hydroformylation en phase organique homogène en présence de composés du rhodium à des concentrations en rhodium de 5 à 100 ppm en poids par rapport au mélange réactionnel homogène ou en présence de composés organiques du phosphore (III) et de composés du rhodium à des concentrations en rhodium de 5 à 500 ppm en poids par rapport au mélange réactionnel homogène et à des températures de 70 à 140°C et des pressions de 5 à 35 MPa par transformation avec du gaz de synthèse en 3(4), 8(9)-bisformyltricyclo [5.2.1.0^{2.6}] décane et on transforme ensuite le 3(4),8(9)-bisformyltricyclo[5.2.1.0^{2.6}] décane en 3(4),8(9)-dihydroxyméthyltricyclo[5.2.1.0²⁻⁶]décane en présence de catalyseurs d'hydrogénation avec de l'hydrogène.

2. Procédé selon la revendication 1, **caractérisé en ce que** le 8(9)-formyltricyclo[5.2.1.0^{2.6}]déc-3-ène obtenu dans la première étape d'hydroformylation est distillé avant son utilisation dans la deuxième étape d'hydroformylation.

3. Procédé selon la revendication 1, **caractérisé en ce qu'**on utilise comme composés organiques du phosphore (III) des triarylphosphines, des trialkylphosphines, des alkylphénylphosphines, des cycloalkylphénylphosphines et des diphosphites organiques.

4. Procédé selon l'une ou plusieurs des revendications 1 à 3, **caractérisé en ce qu'**on utilise dans la première étape d'hydroformylation, comme composés organiques solubles dans l'eau du phosphore (III) des triarylphosphines sulfonées de formule générale (I) 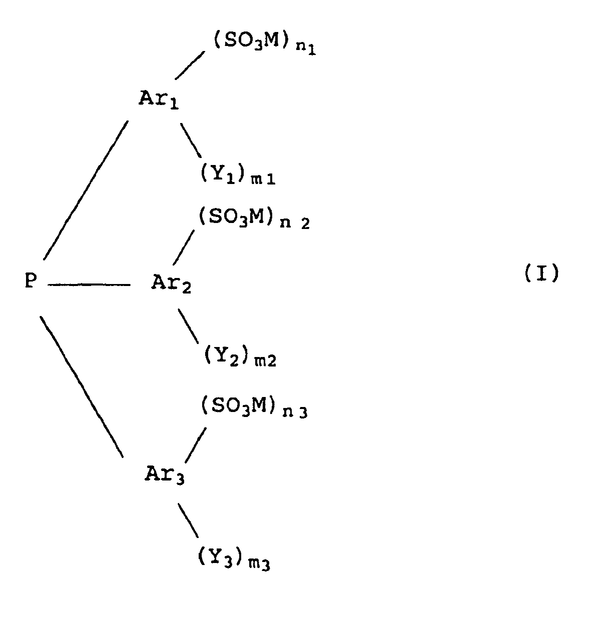 dans laquelle Ar¹, Ar² et Ar³ signifient des groupes aryle identiques ou différents comprenant 6 à 14 atomes de carbone, les substituants Y₁, Y₂ et Y₃ signifient des radicaux alkyle ou alcoxy identiques ou différents, linéaires ou ramifiés comprenant 1 à 4 atomes de carbone, le chlore, le brome, le groupe hydroxyle, cyanure ou nitro, en outre le groupe amino de formule NR¹R², où les substituants R¹ et R² sont identiques ou différents et représentent hydrogène, des groupes alkyle linéaires ou ramifiés comprenant 1 à 4 atomes de carbone, dans laquelle M représente lithium, sodium, potassium, magnésium, calcium ou baryum, dans laquelle m₁, m₂ et m₃ sont identiques ou différents et signifient des nombres entiers de 0 à 5, dans laquelle n₁, n₂ et n₃ sont identiques ou différents et représentent des nombres entiers de 0 à 3, au moins un des nombres n₁, n₂ et n₃ étant égal ou supérieur à 1.

5. Procédé selon la revendication 4, **caractérisé en ce que** Ar₁, Ar₂, Ar₃ signifient à chaque fois un groupe phényle, m₁, m₂, m₃ valent 0, n₁, n₂, n₃ valent 0 ou 1 et n₁+n₂+n₃ valent ensemble 1 à 3, et que les groupes sulfonate se trouvent en position méta.

6. Procédé selon l'une ou plusieurs des revendications 1 à 3, **caractérisé en ce que** dans la première étape d'hydroformylation, on utilise comme composés organiques solubles dans l'eau du phosphore (III) des diphosphines sulfonées de formule générale (II) 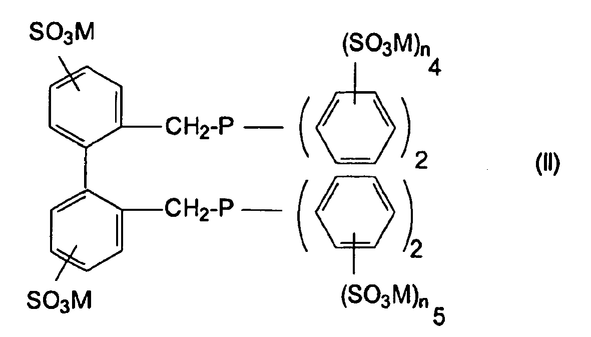 dans laquelle n₄ et n₅ représentent, indépendamment l'un de l'autre 0 ou 1, les diphosphines sulfonées de formule générale (II) contenant jusqu'à six groupes SO₃M, et M signifiant ammonium, un métal monovalent ou l'équivalent d'un métal polyvalent.

7. Procédé selon l'une ou plusieurs des revendications 1 à 3, **caractérisé en ce que** dans la première étape d'hydroformylation, on utilise comme composés organiques solubles dans l'eau du phosphore (III) des diphosphines sulfonées de formule générale (III) 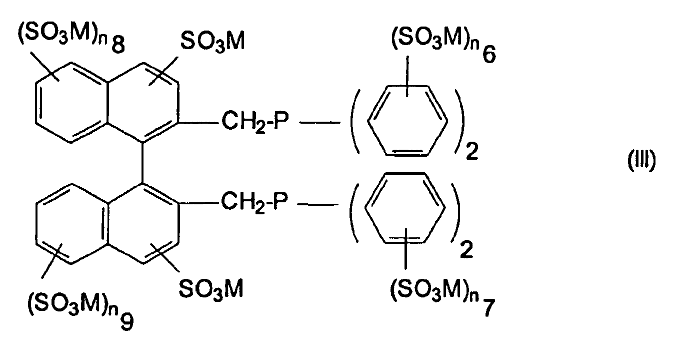 dans laquelle n₆, n₇, n₈ et n₉ représentent, indépendamment l'un de l'autre 0 ou 1, les diphosphines sulfonées de formule générale (III) contenant quatre à huit groupes SO₃M, et M signifiant ammonium, un métal monovalent ou l'équivalent d'un métal polyvalent.

8. Procédé selon l'une ou plusieurs des revendications 1 à 7, **caractérisé en ce que** dans la première étape d'hydroformylation, la température est de 100 à 150°C, de préférence de 110 à 140°C, et la pression est de 1 à 6 MPa, de préférence de 1,5 à 5 MPa.

9. Procédé selon l'une ou plusieurs des revendications 1 à 8, **caractérisé en ce que** dans la deuxième étape d'hydroformylation, la température est de 80 à 130°C, de préférence de 90 à 120°C, et la pression est de 10 à 30 MPa, de préférence de 20 à 30 MPa.

10. Procédé selon l'une ou plusieurs des revendications 1 à 9, **caractérisé en ce que** dans la première étape d'hydroformylation, la concentration en rhodium est de 50 à 800 ppm en poids et en particulier de 100 à 600 ppm en poids, à chaque fois par rapport à la solution aqueuse de catalyseur.

11. Procédé selon l'une ou plusieurs des revendications 1 à 10, **caractérisé en ce que** dans la première étape d'hydroformylation, on utilise, par mole de rhodium, 10 à 300 moles, en particulier 50 à 150 moles, de phosphore sous forme du composé organique soluble dans l'eau du phosphore.

12. Procédé selon l'une ou plusieurs des revendications 1 à 11, **caractérisé en ce que** dans la deuxième étape d'hydroformylation, en présence des composés organiques du phosphore (III), la concentration en rhodium est de 10 à 500 ppm en poids et en particulier de 20 à 500 ppm en poids, à chaque fois par rapport au mélange réactionnel homogène et on utilise par mole de rhodium 5 à 200 moles, de préférence 10 à 100 moles de phosphore sous forme de composés organiques du phosphore.
